# EUROPEAN PATENT APPLICATION

(11) **EP 0 582 450 A2**
(43) Date of publication of application: **09.02.1994**
(21) Application number: 93306099.8
(22) Date of filing: 02.08.1993
(51) Int. Cl.: C12P 21/08, C07K 7/16, C12N 5/18

(54) **Anti-oxytocin receptor antibodies and methods for their production**

(30) Priority: 03.08.1992 JP 206854/92
(71) Applicant: ROHTO PHARMACEUTICAL CO., LTD., Ikuno-ku Osaka-shi Osaka-fu (JP)
(72) Inventor: Saji, Fumitaka, Nishinomiya-shi, Hyogo (JP); Azuma, Chihiro, Osaka-shi, Osaka (JP); Kimura, Tadashi, Suita-shi, Osaka (JP)
(74) Representative: Ritter, Stephen David

(57) **Abstract**

Anti-oxytocin receptor antibodies, characterized in that they recognize, as an epitope, a polypeptide or variant thereof corresponding to the extracellular or intracellular region of an oxytocin receptor.

## Description

The present invention relates to antibodies against a receptor for a posterior pituitary hormone, oxytocin, and methods for their production.

There is strong support for the action of oxytocin as a physiological initiator of delivery in numerous species of mammals, including humans, but the mechanism by which oxytocin accelerates contraction of uterine muscle has not yet been clarified in sufficient detail. An analysis of the diverse research conducted recently on the mechanism of this action indicates that oxytocin manifests this effect in part by inducing direct contractions of the tunica muscularis of the uterus and in part by increasing the synthesis and release of contractile prostaglandins from the endometrium and deciduous membrane. Moreover, initiation of the process that leads to delivery is considered attributable to increased sensitivity of uterine muscle to oxytocin; i.e., initiation of the process leading to delivery is partially attributable to an increase in oxytocin receptors in uterine muscle.

The management of premature birth is a vital issue in the field of obstetrics. It is known that there is an increased number of oxytocin receptors in cases of premature birth or imminent abortion, and in the clinical setting, there is a need for the capability to predict premature birth by measuring the expression level of oxytocin receptors and for developing drugs to suppress labour pains in premature birth. Oxytocin is also known to have lactogenic and hypertensive effects. However, the mode of expression of oxytocin receptors in target tissues remains unclear, so these actions are not yet understood in sufficient detail. Moreover, with regard to another aspect of oxytocin, there are reports that levels of the substance in the blood are elevated in males at the time of ejaculation, and that oxytocin receptors are expressed in rat hippocampus tissue. However, the series of biological responses between oxytocin and oxytocin receptors has not yet been fully explained, and further clarification is needed in this area. With respect to the above clinical and research requirements, one possibility is the use of anti-oxytocin receptor antibodies that specifically recognize oxytocin receptors. Moreover, Kimura et al. recently cloned the cDNA of oxytocin receptors (Nature, 356, 526-529 (1992)) and clarified its primary structure. Based on this primary structure, these receptors were presumed to have the sevenfold-membrane-penetration-type secondary structure typical of
G-protein-binding-type receptors. The development of anti-oxytocin receptor antibodies is desirable for the purification and identification of recombinant oxytocin receptors produced on the basis of these results, as well as for the rapid detection and identification of oxytocin receptors present in oxytocin-receptor-manifesting tissue.

There have as of yet, however, been no reports on antibodies which recognize oxytocin receptors.

Accordingly, the object of the present invention is to provide antibodies against oxytocin receptors.

In order to achieve the above object, the present invention is comprised of: anti-oxytocin receptor antibodies against human oxytocin receptors; a hybridoma which produces monoclonal antibodies that have the above-mentioned properties; a method for the preparation of hybridomas that produce antibodies, in which mammals are sensitized using human oxytocin receptor antigen, immunocytes are harvested from said mammals, said immunocytes are caused to fuse with myeloma cells, and the antibodies recognize human oxytocin receptors from said fused cell strain; a method for the production of anti-human oxytocin receptor monoclonal antibodies in which the aforementioned hybridoma is cultured and monoclonal antibodies are harvested from said culture; and a method for the production of anti-human oxytocin receptor polyclonal antibodies in which mammals or chickens are subjected to immune sensitization with human oxytocin receptor antigen and polyclonal antibodies that recognize human oxytocin receptors are harvested from said animals or egg yolks.

The present invention relates to anti-oxytocin receptor antibodies which recognize, as an epitope, a polypeptide or variant thereof that corresponds to the extracellular or intracellular region of an oxytocin receptor.

The present invention is comprised of: an anti-oxytocin receptor antibody, characterized in that it recognizes a polypeptide corresponding to the extracellular region of an oxytocin receptor, and in that it recognizes, as an epitope, a polypeptide or variant thereof that has all or part of the amino acid sequence represented in Sequence No. 1; and an anti-oxytocin receptor antibody, characterized in that it recognizes a polypeptide corresponding to the intracellular region of an oxytocin receptor, and in that it recognizes, as an epitope, a polypeptide or variant thereof that has all or part of the amino acid sequence represented in Sequence No. 7. Furthermore, the present invention is also comprised of: a method of producing an anti-oxytocin receptor antibody comprising the preparation of fused cells from mammalian spleen cells and myeloma cells immunized using, as a hapten, a polypeptide or variant thereof that corresponds to the extracellular region of an oxytocin receptor and has all or part of the amino acid sequence represented in Sequence No. 1, culturing said fused cells, and harvesting anti-oxytocin receptor antibodies which recognize, as an epitope, a polypeptide or variant thereof that corresponds to the extracellular region of the oxytocin receptor and has all or part of the amino acid sequence represented in Sequence No. 1; a method of producing an anti-oxytocin receptor antibody comprising the preparation of mammalian serum or chicken egg yolks immunized using, as a hapten, a polypeptide or variant thereof that corresponds to the extracellular region of an oxytocin receptor and has all or part of the amino acid sequence represented in Sequence No. 1, purifying said prepared solution, and harvesting anti-oxytocin receptor antibodies which recognize, as an epitope, a polypeptide or variant thereof that corresponds to the extracellular region of the oxytocin receptor and has all or part of the amino acid sequence represented in Sequence No. 1; a method of producing an anti-oxytocin receptor antibody comprising the preparation of fused cells from mammalian spleen cells and myeloma cells immunized using, as a hapten, a polypeptide or variant thereof that corresponds to the intracellular region of an oxytocin receptor and has all or part of the amino acid sequence represented in Sequence No. 7, culturing said fused cells, and harvesting anti-oxytocin receptor antibodies which recognize, as an epitope, a polypeptide or variant thereof that corresponds to the intracellular region of the oxytocin receptor and has all or part of the amino acid sequence represented in Sequence No. 7; and a method of producing an anti-oxytocin receptor antibody comprising the preparation of mammalian serum or chicken egg yolks immunized using, as a hapten, a polypeptide or variant thereof that corresponds to the intracellular region of an oxytocin receptor and has all or part of the amino acid sequence represented in Sequence No. 7, purifying said prepared solution, and harvesting anti-oxytocin receptor antibodies which recognize, as an epitope, a polypeptide or variant thereof that corresponds to the intracellular region of the oxytocin receptor and has all or part of the amino acid sequence represented in Sequence No. 7.

### Examples

The antibodies of the present invention include those which recognize, as an epitope, a polypeptide of the extracellular region of an oxytocin receptor and those which recognize, as an epitope, a polypeptide of the intracellular region of an oxytocin receptor. Specific examples of such polypeptides include the polypeptides of the first extracellular region and the third intracellular region.

Any immunogens may be used to produce the antibodies of the present invention, provided that they have a macromolecular carrier bonded to the hapten. Specific examples of such haptens include polypeptides obtained by chemical synthesis of part of the amino acid sequence of the cDNA translation region of the oxytocin receptors presented by Kimura et al., as mentioned above. These should preferably be polypeptides of the extracellular or intracellular region of said receptors, with polypeptides of the first extracellular region or third intracellular region of said receptors being particularly desirable, and should be polypeptides or variants thereof that have all or part of the amino acid sequences represented in Sequence Nos. 1 or 7. In this case, one or several amino acid residues of the aforementioned polypeptides may be substituted, deleted, or added. Moreover, any macromolecular carrier is suitable, provided that it enhances the immune response; specific examples of such carriers include keyhole limpet haemocyanin (KLH) and ovalbumin (OVA). Moreover, all or part of the aforementioned cDNA may be integrated into an expression vector that has an appropriate promoter, and recombinant protein obtained by inducing expression in E. coli or mammalian cells may be used as an immunogen. Any of these immunogens may be used to prepare hybridomas that produce monoclonal antibodies or to prepare polyclonal antibodies.

Hybridoma preparation may be carried out by any common method. For example, mammals such as mice may be immunized with any of the aforementioned immunogens, spleen cells may be removed from the immunized animals, and these cells may be fused with established myeloma cells. A hybridoma producing monoclonal antibodies that have the desired reactivity is then cloned.

In the preparation of monoclonal antibodies, as we have mentioned above, one possible method is to culture the cloned hybridoma on an appropriate culture medium and harvest the monoclonal antibodies from the culture supernatant. Another method is to introduce the aforementioned hybridoma into the peritoneal cavity of the animals and to purify and isolate the antibodies from the ascites fluid that is obtained.

Production of polyclonal antibodies may also be carried out by a common method, such as by immunizing mice, rabbits, sheep, goats, chickens, etc. with the aforementioned immunogens and purifying the serum of these animals, or in the case of chickens, the egg yolks.

The antibodies in the aforementioned culture supernatant, ascites fluid, serum, or egg yolk may be concentrated by a common method such as by salting out with ammonium sulphate, or in the case of antibodies in egg yolk, by concentration using the well-known carrageenan treatment method. Purification may be performed using a combination of an appropriate ion-exchange agent and a gel filtration agent, or may be performed by affinity chromatography using protein A, protein G, or the aforementioned antigens as ligands.

The aforementioned hybridoma preparation methods, monoclonal antibody production methods, polyclonal antibody preparation methods, and antibody production methods are all methods well-known to one having ordinary skill in the art.

In the following, we shall present examples in order to further explain the present invention, but the invention is not limited to these examples.

### Example 1: Preparation of Monoclonal Antibodies Against Human Oxytocin Receptors

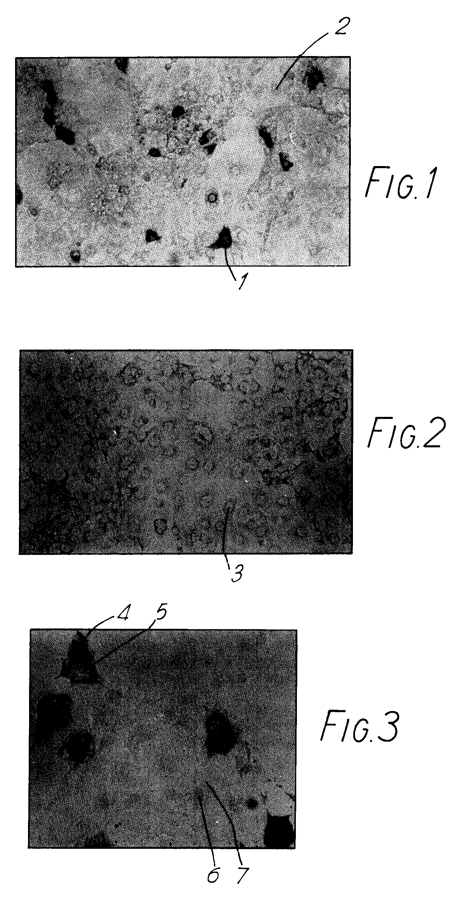

In order to prepare mouse monoclonal antibodies against human oxytocin receptors, a peptide consisting of a cysteine residue added to the N terminal of the peptide represented in Sequence No. 1, which is composed of 19 amino acids and corresponds to part of the region presumed to be the first extracellular region, was synthesized as an immunogen by a common method. Using the method presented by Hashida et al. *(Journal of Applied Biochemistry,* 6, 56-63, 1984), this peptide was caused to bind to keyhole lipid haemocyanin (KLH, Calbiochem Co.) and to albumin, chicken egg grade V (Ovalbumin, Sigma Co.). 50 µg/0.1 ml of H₂O (pure water) of this peptide-KLH combination was subcutaneously injected into BALB/C mice, together with 0.1 ml of Freund's complete adjuvant (FCA, Difco Laboratories, Inc.) to produce immunization. After 10 days, 5 µg/0.2 ml (pure water) of said peptide-KLH combination was administered into the peritoneal cavity for additional immunization, and after 3 days, spleen cells were taken from the mice. By means of a common method using polyethylene glycol, these spleen cells were fused with the myeloma cells P3X63Ag8U1 (Dainippon Pharmaceutical Co., Ltd.), and cloning was performed using the limiting dilution analysis method. Evaluation of the antibody titre of the cloned cells was performed using the common ELISA method, by adding the hybridoma culture supernatant to a microtiter plate with the aforementioned peptide-OVA combination solidified on it, and then measuring the amount of mouse antibodies binding to the peptide-OVA combination following the reaction. A hybridoma producing cloned antibodies that recognize the peptide-OVA combination obtained by this method was cultured in GIT culture medium (Nihon Seiyaku Co., Ltd.), and antibodies were obtained from the culture supernatant through crude purification by ordinary salting out with ammonium sulphate. The segment extending from the first BamHI recognition site upstream from the translation region of the plasmid pOTR (*Nature*, 356, 526-529, 1992) containing the cDNA encoding the aforementioned human oxytocin receptors to the first PstI recognition site downstream from the translation region was then cut, removed, and made into a smooth terminal by a common method, and a recombinant DNA molecule with this segment introduced at the multi-cloning site of the mammalian cell expression vector pRc/CMV (Invitrogen Corporation, U.S.A.) was introduced into COS1 cells (Dainippon pharmaceutical Co., Ltd.) by the common calcium phosphate method. Confirmation of transient expression of human oxytocin receptors of the COS1 cells 3 days after introduction was effected by common immune staining using the various antibodies obtained above (antibody concentration 10 µg/ml). Specifically, cells cultured on a glass slide were fixed for 10 minutes with acetic acid/methanol (1 : 3, V/V). In order to eliminate their intrinsic peroxidase activity, they were then treated for 30 minutes with methanol containing 0.3% hydrogen peroxide, and ordinary immune staining was performed with the Vectastain^{(TM)} ABC Kit (Vector Laboratories, U.S.A.), using the aforementioned monoclonal antibodies (antibody concentration 10 µm/ml) as primary antibodies. A mixture of equivalent amounts of 0.02% hydrogen peroxide and 0.1% 3,3'-diaminobenzidine tetrahydrochloride (DAB) (Nakarai Kagaku Yakuhin K.K.) solution (0.1 M tris-buffer solution, prepared to a pH of 7.2) was used as a colour substrate solution. As a result, it was possible to introduce said recombinant DNA molecule and to detect antibodies (YA-1 antibodies), which with immune staining stain only those cells that express oxytocin receptors (see Figures 1 and 2). Figure 1 is a sketch of a micrograph (magnified 100×) of the results of immune staining of COS1 cells with recombinant DNA introduced using the monoclonal antibodies of the present invention, and Figure 2 is a sketch of a micrograph (magnified 100x) of the results of immune staining of COS1 cells without recombinant DNA introduced using the monoclonal antibodies of the present invention.

The micrograph represented in Figure 1 shows that the COS1 cells that temporarily express oxytocin receptors (1) are stained brown, while the COS1 cells which do not temporarily express oxytocin receptors (2) are not stained (transparent). Moreover, in the micrograph represented in Figure 2, one can see that the COS1 cells which do not express oxytocin receptors (3) are not stained (transparent). The hybridoma that produces these antibodies was designated mouse/mouse hybridoma YA-1. This hybridoma YA-1 was deposited on July 28, 1992 with the Fermentation Research Institute of the Agency of Industrial Science and Technology as Bacterial Deposit No. 13095 (FERM P-13095).

### Example 2: Preparation of Polyclonal Antibodies Against Human Oxytocin Receptors

In order to prepare polyclonal antibodies against human oxytocin receptors, a peptide consisting of a cysteine residue added to the N terminal of the peptide represented in Sequence No. 7, which is composed of 15 amino acids and corresponds to part of the region presumed to be the third intracellular region, was synthesized as an immunogen by a common method. As was the case in Example 1, this peptide was caused to bind to OVA (Sigma Co.). Approximately 0.5 mg/1 ml (pure water) of this peptide-OVA combination was administered to Japanese white rabbits together with 1 ml of Freund's complete adjuvant (Difco Laboratories, Inc.) for initial immunization, 6 subsequent immunizations were performed once weekly thereafter (for a total of 7 immunizations) with the same amount of peptide-OVA combination together with Freund's incomplete adjuvant (FIA, Difco Laboratories, Inc.), and whole blood samples were taken. The serum was isolated using a common method, and the serum obtained was subjected to affinity purification using the Protein A sepharose CL-4B column (Pharmacia LKB Biotechnology).

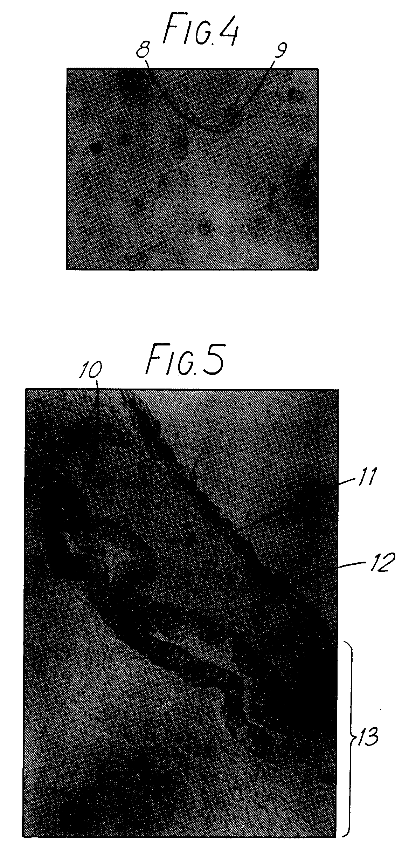

Figures 3 and 4 show that polyclonal antibodies produced in this manner specifically recognize oxytocin receptors. Specifically, as was the case in Example 1, the mammalian expression vector pRc/CMV containing the cDNA encoding the human oxytocin receptors obtained in Example 1 was introduced into COS1 cells. With respect to its transient expression, ordinary immune staining was performed using the aforementioned antibodies (antibody concentration: 10 µg/ml) and, as was the case in Example 1, the Vectastain ABC Kit (Vector Laboratories). A mixture of equivalent amounts of 0.02% hydrogen peroxide and 0.1% DAB (Nakarai Kagaku Yakuhin K.K.) solution (0.1 M tris-buffer solution, adjusted to a pH of 7.2) was used as a colour substrate solution. After this, ordinary haematoxylin staining (Mayer's haematoxylin, Takefu Kagaku Yakuhin K.K.) was performed. The results showed that when the aforementioned polyclonal antibodies were used, stained cells were observed (see Figure 3), but with antibodies obtained by immunization with OVA alone, stained cells were not observed (see Figure 4).

Figure 3 is a sketch of a micrograph (magnified 200x) of the results of immune staining of COS1 cells expressing oxytocin receptors using the polyclonal antibodies of the present invention, and Figure 4 is a sketch of a micrograph (magnified 200x) of the results of immune staining of COS1 cells expressing oxytocin receptors using antibodies obtained by immunization with OVA alone. The micrograph represented in Figure 3 shows that the COS1 cells expressing oxytocin receptors (4) are stained brown by the antibodies of the present invention, while the micrograph represented in Figure 4 shows that staining does not occur (transparent) with the antibodies obtained by immunization with OVA alone. Moreover, haematoxylin staining of the nuclei (5, 6, and 9) of the COS1 cells produces a blue colour.

### Example 3: Preparation of Polyclonal Antibodies Against Human Oxytocin Receptors

Using the peptide-OVA combination prepared in Example 1 as an immunogen, Japanese white rabbits were immunized by the same method as in Example 2, and the serum obtained was subjected to affinity purification using the Protein A sepharose CL-4B column (Pharmacia LKB Biotechnology).

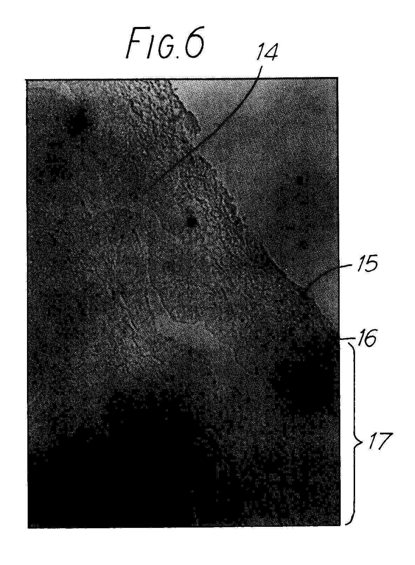

Human uterine muscle obtained by chance during the ovulation period (in the form of a pathology specimen from a patient who had to undergo a total hysterectomy due to a hysteromyoma) was rapidly frozen to -70° in liquid nitrogen. Frozen sections of this tissue were prepared using a cryostat (Tissue-Tek II^{(TM)}, Miles Co.), and ordinary immunohistological staining was performed. Specifically, fragments adhering to the glass slide were fixed for 10 minutes with acetone, and in order to eliminate their intrinsic peroxidase activity, they were then treated for 30 minutes with methanol containing 0.3% hydrogen peroxide. After this, ordinary immunohistological staining was performed with the aforementioned polyclonal antibodies (antibody concentration 20 µg/ml) as primary antibodies, using the Vectastain ABC Kit (Vector Laboratories). A mixture of equivalent amounts of 0.02% hydrogen peroxide and 0.1% DAB (Nakarai Kagaku Yakuhin K.K.) solution (0.1 M tris-buffer solution, adjusted to a pH of 7.2) was used as a colour substrate solution. Figures 5 and 6 show that oxytocin receptors that are expressed in epithelial cells of the endometrium during the ovulation period were stained only when polyclonal antibodies prepared as described above were used as primary antibodies (see Figure 5).

Figure 5 is a sketch of a micrograph (magnified 200x) of the results of immunohistological staining of human endometrial tissue using the polyclonal antibodies of the present invention, and Figure 6 is a sketch of a micrograph (magnified 200x) of the results of immunohistological staining of antibodies obtained by immunization with OVA alone.

The micrograph represented in Figure 5 shows that the endometrial epithelial cells (10, 11) were stained brown, while the endometrial interstitium (12) was not stained (transparent). Moreover, one can see from the micrograph represented in Figure 6 that the endometrial epithelial cells (14, 15) were not stained (transparent).

The antibodies of the present invention consist of those which specifically recognize the extracellular region of an oxytocin receptor and those which specifically recognize the intracellular region of an oxytocin receptor. The former antibodies may be used as agents to inhibit the physiological activity of oxytocin in order to block specific binding with respect to oxytocin receptors, for instance, as agents which regulate the time of delivery, i.e., agents which inhibit labour pains in premature birth. Moreover, seeing as oxytocin receptors are believed to be receptors of the G-protein-binding type, the latter antibodies may be used in the same way to inhibit oxytocin activity by blocking binding to G-protein receptors.

In further embodiments of the invention antibodies are prepared that recognize as an epitope the specific sequences shown in any of Sequences No.s 1,2,3,4,5,6 and 7 or parts thereof using methods analagous to those described above in relation to Sequences No.s 1 and 7.

Of course, these antibodies may also be used in the fields of obstetrics and gynaecology as reagents for immune staining of pathological tissues such as from the mammaries, uterine muscle, endometrium, deciduous membrane, amnion, and so forth. Moreover, seeing as proteins which can bind to oxytocin in the hippocampus tissue of rats are known to exist, these antibodies may also be used in neuroscientific research. By using the antibodies of the present invention, it is therefore possible to further clarify the mechanism of the series of biological responses between oxytocin and oxytocin receptors, a problem that has hitherto remained largely unsolved, and this should be an aid in the development of pharmaceuticals in this field.

Furthermore, with respect to the development of oxytocin antagonists using transformants that can be obtained by introducing the cDNA of the oxytocin receptors presented by Kimura et al. (described above) on an appropriate expression vector and carrying out phenotypic transformation of mammalian cells, the antibodies of the present invention can provide a more favourable screening system.

SEQUENCE LISTING

(1) GENERAL INFORMATION:

(i) APPLICANT:
(A) NAME: ROHTO PHARMACEUTICAL CO., LTD
(B) STREET: 8-1, TATSUMI NISHI 1-CHOME, IKUNO-KU,
OSAKA-SHI
(C) CITY: OSAKA
(E) COUNTRY: JAPAN
(F) POSTAL CODE (ZIP): NONE

(ii) TITLE OF INVENTION: ANTI-OXYTOCIN RECEPTOR ANTIBODIES AND METHODS
FOR THEIR PRODUCTION

(iii) NUMBER OF SEQUENCES: 7

(iv) COMPUTER READABLE FORM:
(A) MEDIUM TYPE: Floppy disk
(B) COMPUTER: IBM PC compatible
(C) OPERATING SYSTEM: PC-DOS/MS-DOS
(D) SOFTWARE: PatentIn Release #1.0, Version #1.25 (EPO)

(2) INFORMATION FOR SEQ ID NO: 1:

(i) SEQUENCE CHARACTERISTICS:
(A) LENGTH: 19 amino acids
(B) TYPE: amino acid
(C) STRANDEDNESS: single
(D) TOPOLOGY: linear

(ii) MOLECULE TYPE: peptide

(iii) HYPOTHETICAL: NO

(iii) ANTI-SENSE: NO

(xi) SEQUENCE DESCRIPTION: SEQ ID NO: 1:

Met Glu Gly Ala Leu Ala Ala Asn Trp Ser Ala Glu Ala Ala Asn Ala
1               5                   10                  15

Ser Ala Ala

(2) INFORMATION FOR SEQ ID NO: 2:

(i) SEQUENCE CHARACTERISTICS:
(A) LENGTH: 19 amino acids
(B) TYPE: amino acid
(C) STRANDEDNESS: single
(D) TOPOLOGY: linear

(ii) MOLECULE TYPE: peptide

(iii) HYPOTHETICAL: NO

(iii) ANTI-SENSE: NO

(xi) SEQUENCE DESCRIPTION: SEQ ID NO: 2:

Ala Asn Ala Ser Ala Ala Pro Pro Gly Ala Glu Gly Asn Arg Thr Ala
1               5                   10                  15

Gly Pro Pro

(2) INFORMATION FOR SEQ ID NO: 3:

(i) SEQUENCE CHARACTERISTICS:
(A) LENGTH: 21 amino acids
(B) TYPE: amino acid
(C) STRANDEDNESS: single
(D) TOPOLOGY: linear

(ii) MOLECULE TYPE: peptide

(iii) HYPOTHETICAL: NO

(iii) ANTI-SENSE: NO

(xi) SEQUENCE DESCRIPTION: SEQ ID NO: 3:

Pro Pro Gly Ala Glu Gly Asn Arg Thr Ala Gly Pro Pro Arg Arg Asn
1               5                   10                  15

Glu Ala Leu Ala Arg
            20

(2) INFORMATION FOR SEQ ID NO: 4:

(i) SEQUENCE CHARACTERISTICS:
(A) LENGTH: 18 amino acids
(B) TYPE: amino acid
(C) STRANDEDNESS: single
(D) TOPOLOGY: linear

(ii) MOLECULE TYPE: peptide

(iii) HYPOTHETICAL: NO

(iii) ANTI-SENSE: NO

(xi) SEQUENCE DESCRIPTION: SEQ ID NO: 4:

Thr Phe Arg Phe Tyr Gly Pro Asp Leu Leu Cys Arg Leu Val Lys Tyr
1               5                   10                  15

Leu Gln

(2) INFORMATION FOR SEQ ID NO: 5:

(i) SEQUENCE CHARACTERISTICS:
(A) LENGTH: 19 amino acids
(B) TYPE: amino acid
(C) STRANDEDNESS: single
(D) TOPOLOGY: linear

(ii) MOLECULE TYPE: peptide

(iii) HYPOTHETICAL: NO

(iii) ANTI-SENSE: NO

(xi) SEQUENCE DESCRIPTION: SEQ ID NO: 5:

Ser Leu Arg Glu Val Ala Asp Gly Val Phe Asp Cys Trp Ala Val Phe
1               5                   10                  15

Ile Gln Pro

(2) INFORMATION FOR SEQ ID NO: 6:

(i) SEQUENCE CHARACTERISTICS:
(A) LENGTH: 12 amino acids
(B) TYPE: amino acid
(C) STRANDEDNESS: single
(D) TOPOLOGY: linear

(ii) MOLECULE TYPE: peptide

(iii) HYPOTHETICAL: NO

(iii) ANTI-SENSE: NO

(xi) SEQUENCE DESCRIPTION: SEQ ID NO: 6:

Ser Val Trp Asp Ala Asn Ala Pro Lys Glu Ala Ser
1               5                   10

(2) INFORMATION FOR SEQ ID NO: 7:

(i) SEQUENCE CHARACTERISTICS:
(A) LENGTH: 15 amino acids
(B) TYPE: amino acid
(C) STRANDEDNESS: single
(D) TOPOLOGY: linear

(ii) MOLECULE TYPE: peptide

(iii) HYPOTHETICAL: NO

(iii) ANTI-SENSE: NO

(xi) SEQUENCE DESCRIPTION: SEQ ID NO: 7:

Ser Ser Val Lys Leu Ile Ser Lys Ala Lys Ile Arg Thr Val Lys
1               5                   10                  15

SEQUENCE TABLES
Sequence No.: 1
Sequence length: 19
Type of sequence: Amino acid
Topology: Straight chain
Kind of sequence: Peptide
Characteristics
of sequence: /Part of the first extracellular
region of the oxytocin receptor.
Sequence
Met Glu Gly Ala Leu Ala Ala Asn Trp Ser Ala Glu Ala Ala Asn
1               5                   10                  15
Ala Ser Ala Ala
19

Sequence No.: 2
Sequence length: 19
Type of sequence: Amino acid
Topology: Straight chain
Kind of sequence: Peptide
Characteristics
of sequence: /Part of the oxytocin receptor.
Sequence
Ala Asn Ala Ser Ala Ala Pro Pro Gly Ala Glu Gly Asn Arg Thr
1               5                   10                  15
Ala Gly Pro Pro
19

Sequence No.: 3
Sequence length: 21
Type of sequence: Amino acid
Topology: Straight chain
Kind of sequence: Peptide
Characteristics
of sequence: /Part of the oxytocin receptor.
Sequence
Pro Pro Gly Ala Glu Gly Asn Arg Thr Ala Gly Pro Pro Arg Arg
1               5                   10                  15
Asn Glu Ala Leu Ala Arg
                20

Sequence No.: 4
Sequence length: 18
Type of sequence: Amino acid
Topology: Straight chain
Kind of sequence: Peptide
Characteristics
of sequence: /Part of the oxytocin receptor.

Sequence
Thr Phe Arg Phe Tyr Gly Pro Asp Leu Leu Cys Arg Leu Val Lys
1               5                   10                  15
Tyr Leu Gln
18

Sequence No.: 5
Sequence length: 19
Type of sequence: Amino acid
Topology: Straight chain
Kind of sequence: Peptide
Characteristics
of sequence: /Part of the oxytocin receptor.
Sequence
Ser Leu Arg Glu Val Ala Asp Gly Val Phe Asp Cys Trp Ala Val
1               5                   10                  15
Phe Ile Gln Pro
19

Sequence No.: 6
Sequence length: 12
Type of sequence: Amino acid
Topology: Straight chain
Kind of sequence: Peptide
Characteristics
of sequence: /Part of the oxytocin receptor.
Sequence
Ser Val Trp Asp Ala Asn Ala Pro Lys Glu Ala Ser
1               5                   10

Sequence No.: 7
Sequence length: 15
Type of sequence: Amino acid
Topology: Straight chain
Kind of sequence: Peptide
Characteristics
of sequence: /Part of the third intracellular
region of the oxytocin receptor.
Sequence
Ser Ser Val Lys Leu Ile Ser Lys Ala Lys Ile Arg Thr Val Lys
1               5                   10                  15

### There now follows a brief description of the figures.

### Figure 1

This is a sketch of a micrograph (magnified 100x) of the results of immune staining of COS1 cells with recombinant DNA introduced using the monoclonal antibodies of Example 1 obtained by immunization with a combination of KLH and the polypeptide represented in Sequence No. 1. 1: Oxytocin receptor expressed COS1 cells (brown). 2: Oxytocin receptor non-expressed COS1 cells (transparent)

### Figure 2

This is a sketch of a micrograph (magnified 100×) of the results of immune staining of COS1 cells without recombinant DNA introduced using the monoclonal antibodies of Example 1 obtained by immunization with a combination of KLH and the polypeptide represented in Sequence No. 1. 3: Oxytocin receptor non-expressed COS1 cells (transparent).

### Figure 3

This is a sketch of a micrograph (magnified 200x) of the results of immune staining of COS1 cells with recombinant DNA introduced using the polyclonal antibodies of Example 2 obtained by immunization with a combination of OVA and the polypeptide represented in Sequence No. 7. 4: Oxytocin receptor expressed COS1 cells (brown). 5, 6: Nuclei (blue). 7: Oxytocin receptor non-expressed COS1 cells (transparent).

### Figure 4

This is a sketch of a micrograph (magnified 200x) of the results of immune staining of COS1 cells with recombinant DNA introduced using the polyclonal antibodies of Example 2 obtained by immunization with OVA alone. 8: Oxytocin receptor non-stained or non-expressed COS1 cells (transparent). 9: Nucleus (blue).

### Figure 5

This is a sketch of a micrograph (magnified 200x) of the results of immunohistological staining of human endometrial tissue using the polyclonal antibodies of Example 3 obtained by immunization with a combination of OVA and the polypeptide represented in Sequence No. 1. 10, 11: Endometrial epithelial cells (brown). 12: Endometrial interstitium (transparent). 13: Endometrial tissue.

### Figure 6

This is a sketch of a micrograph (magnified 200x) of the results of immunohistological staining of human endometrial tissue using the polyclonal antibodies of Example 3 obtained by immunization with OVA alone. 14, 15: Endometrial epithelial cells (transparent). 16: Endometrial interstitium (transparent). 17: Endometrial tissue.

## Claims

1. An anti-oxytocin receptor antibody.

2. An anti-oxytocin receptor antibody according to claim 1, characterized in that it recognizes, as an epitope, a polypeptide corresponding to the extracellular or intracellular region of an oxytocin receptor or it recognizes as an epitope a variant having at least 80% homology with said polypeptide.

3. An anti-oxytocin receptor antibody according to claim 1 or 2 characterized in that it recognizes a polypeptide corresponding to the extracellular region of an oxytocin receptor, and in that it recognizes, as an epitope, a polypeptide that has the amino acid sequence represented in Sequence No. 1 or a fragment thereof containing at least six contiguous amino acids.

4. An anti-oxytocin receptor antibody according to Claim 3, characterized in that it recognizes, as an epitope, a molecule comprising a polypeptide having the amino acid sequence represented in Sequence No. 1, in which zero, one, two, three or four amino acid residues have been substituted, deleted, or added.

5. A method of producing an anti-oxytocin receptor antibody comprising the preparation of fused cells from mammalian spleen cells and myeloma cells immunized using, as a hapten, a polypeptide that corresponds to the extracellular region of an oxytocin receptor and has the amino acid sequence represented in Sequence No. 1 or a fragment thereof containing at least six contiguous amino acids, culturing said fused cells, and harvesting anti-oxytocin receptor antibodies which recognize, as an epitope, a polypeptide that corresponds to the extracellular region of the oxytocin receptor and has the amino acid sequence represented in Sequence No. 1 or a fragment thereof containing at least six contiguous amino acids.

6. A method of producing an anti-oxytocin receptor antibody according to Claim 5, characterized in that the mammals used are mice or rats.

7. A method of producing an anti-oxytocin receptor antibody comprising the preparation of mammalian serum or chicken egg yolks immunized using, as a hapten, a polypeptide that corresponds to the extracellular region of an oxytocin receptor and has the amino acid sequence represented in Sequence No. 1 or a fragment thereof containing at least six contiguous amino acids, purifying said prepared solution, and harvesting anti-oxytocin receptor antibodies which recognize, as an epitope, a polypeptide that corresponds to the extracellular region of the oxytocin receptor and has the amino acid sequence represented in Sequence No. 1 or a fragment thereof containing at least six contiguous amino acids.

8. An anti-oxytocin receptor antibody according to claim 1 or 2 characterized in that it recognizes a polypeptide corresponding to the intracellular region of an oxytocin receptor, and in that it recognizes, as an epitope, a polypeptide that has the amino acid sequence represented in Sequence No. 7 or a fragment thereof containing at least six contiguous amino acids.

9. An anti-oxytocin receptor antibody according to Claim 8, characterized in that it recognizes, as an epitope, a molecule comprising a polypeptide having the amino acid sequence represented in Sequence No. 7 in which zero, one, two or three amino acid residues have been substituted, deleted, or added.

10. A method of producing an anti-oxytocin receptor antibody comprising the preparation of fused cells from mammalian spleen cells and myeloma cells immunized using, as a hapten, a polypeptide that corresponds to the intracellular region of an oxytocin receptor and has the amino acid sequence represented in Sequence No. 7 or a fragment thereof containing at least six contiguous amino acids, culturing said fused cells, and harvesting anti-oxytocin receptor antibodies which recognize, as an epitope, a polypeptide that corresponds to the intracellular region of the oxytocin receptor and has the amino acid sequence represented in Sequence No. 7 or a fragment thereof containing at least six contiguous amino acids.

11. A method of producing an anti-oxytocin receptor antibody according to Claim 10, characterized in that the mammals used are mice or rats.

12. A method of producing an anti-oxytocin receptor antibody comprising the preparation of mammalian serum or chicken egg yolks immunized using, as a hapten, a polypeptide that corresponds to the intracellular region of an oxytocin receptor and has the amino acid sequence represented in Sequence No. 7 or a fragment thereof containing at least six contiguous amino acids, purifying said prepared solution, and harvesting anti-oxytocin receptor antibodies which recognize, as an epitope, a polypeptide that corresponds to the intracellular region of the oxytocin receptor and has the amino acid sequence represented in Sequence No. 7 or a fragment thereof containing at least six contiguous amino acids.

13. An anti-oxytocin receptor antibody according to claim 1 or 2 characterized in that it recognizes, as an epitope, a polypeptide that has the amino acid sequence represented in any one of Sequences No.s 2-6 or a fragment thereof containing at least six contiguous amino acids.

14. A method of producing an anti-oxytocin receptor antibody comprising the preparation of fused cells from mammalian spleen cells and myeloma cells immunized using, as a hapten, a polypeptide that has the amino acid sequence represented in any one of Sequences No.s 2-6 or a fragment thereof containing at least six contiguous amino acids, culturing said fused cells, and harvesting anti-oxytocin receptor antibodies which recognize, as an epitope, a polypeptide that has the amino acid sequence represented in any one of Sequences No.s 2-6 or a fragment thereof containing at least six contiguous amino acids.

15. A method of producing an anti-oxytocin receptor antibody comprising the preparation of mammalian serum or chicken egg yolks immunized using, as a hapten, a polypeptide that has the amino acid sequence represented in any one of Sequences No.s 2-6 or a fragment thereof containing at least six contiguous amino acids, purifying said prepared solution, and harvesting anti-oxytocin receptor antibodies which recognize, as an epitope, a polypeptide that has the amino acid sequence represented in any one of Sequences No.s 2-6 or a fragment thereof containing at least six contiguous amino acids.

16. A hybridoma capable of producing an antibody according to any of claims 1-4 or 8-9 or 13.

17. Hybridoma YA-1 which has been deposited with the Fermentation Research Institute and has been accorded Accession number FERM P-13095.

18. A method for detecting the presence of oxytocin receptors in an analyte comprising:
(i) incubating an analyte suspected of containing oxytocin receptor polypeptides with one or more antibodies according to any of claims 1-4, 8-9 or 13;
(ii) detecting the presence of oxytocin receptor-antibody immune complexes.
